# EUROPEAN PATENT APPLICATION

(11) **EP 4 618 010 A1**
(43) Date of publication of application: **17.09.2025**
(21) Application number: 25161048.1
(22) Date of filing: 28.02.2025
(51) Int. Cl.: G06T 7/00

(54) **IMAGE PROCESSING APPARATUS, RADIOGRAPHY SYSTEM, AND PROGRAM**

(30) Priority: 11.03.2024 JP 2024037653
(71) Applicant: FUJIFILM Corporation, Tokyo Tokyo 106-8620 (JP)
(72) Inventor: KANEKO, Yasuhiko, Kanagawa (JP)
(74) Representative: Dehns Germany Partnerschaft mbB

(57) **Abstract**

Provided are an image processing apparatus, a radiography system, and a program that can detect a calcification lesion from a radiation image of a breast in a state in which an influence of noise is reduced, as compared with the related art.

An image processing apparatus includes an image acquisition unit (30) that acquires a radiation image obtained by imaging a breast with radiation, a normalization unit (32) that normalizes a rate of change in a brightness gradient in the acquired radiation image using a variation degree that indicates a degree of variation in a local region for each local region, and a calcification detection unit (33) that detects a position of a calcification lesion in the radiation image by performing threshold value processing using a predetermined threshold value on the normalized rate of change in the brightness gradient.

## Description

### BACKGROUND OF THE INVENTION

### 1. Field of the Invention

The present disclosure relates to an image processing apparatus, a radiography system, and a program.

### 2. Description of the Related Art

In recent years, image diagnosis using a radiography apparatus (called mammography) for capturing an image of a breast has attracted attention in order to promote early detection of breast cancer. Further, in the mammography, tomosynthesis imaging has been proposed which moves a radiation source, irradiates the breast with radiation at a plurality of radiation source positions to acquire a plurality of projection images, and reconstructs the plurality of acquired projection images to generate tomographic images in which desired tomographic planes have been emphasized. In the tomosynthesis imaging, the plurality of projection images are acquired by imaging the breast at the plurality of radiation source positions by moving the radiation source in parallel with a radiation detector or moving the radiation source to draw a circle or an elliptical arc in accordance with characteristics of an imaging apparatus and a required tomographic image, and the projection images are reconstructed using a back projection method such as a simple back projection method or a filtered back projection method or a sequential reconstruction method. Thereby, a tomographic image is generated.

The tomographic images are generated in a plurality of tomographic planes of the breast, which makes it possible to separate structures that overlap each other in the depth direction in which the tomographic planes are arranged in the breast. Therefore, it is possible to find an abnormal part such as a lesion that has been difficult to detect in a two-dimensional image (hereinafter, referred to as a simple two-dimensional image) acquired by simple imaging according to the related art which irradiates a subject with radiation in a predetermined direction.

As a technology using the tomographic images in this way, JP2014-128716A discloses a technology of generating a pseudo two-dimensional image (hereinafter, referred to as a synthesized two-dimensional image) corresponding to a simple two-dimensional image by synthesizing a plurality of tomographic images having different distances (positions in a height direction) from a detection surface of a radiation detector to a radiation source, using an addition method, an averaging method, a maximum intensity projection method, a minimum intensity projection method, or the like, the tomographic images being acquired by tomosynthesis imaging.

By the way, in the radiation image of the breast, such as the synthesized two-dimensional image described above, it is easy to detect a calcification lesion having a large size (hereinafter, also referred to as a "calcification lesion"), but it is significantly difficult to detect a calcification lesion having a relatively small size because the influence of noise has a large effect.

As a technique that can be applied to solve this problem, JP2006-325640A discloses a method of displaying abnormal shadow candidates that are suspected to be true positive abnormal shadows and/or abnormal shadow candidates with low visibility, and providing only detection information of the abnormal shadow candidates, with the aim of preventing a doctor from overlooking the abnormal shadow candidates and improving the efficiency of the image interpretation work.

The display method comprises a detection step of performing image analysis on a medical image to detect an abnormal shadow candidate, an extraction step of extracting an abnormal shadow candidate to be displayed among the detected abnormal shadow candidates, and a display step of displaying detection information of the extracted abnormal shadow candidate.

In addition, JP2010-000130A discloses an image display apparatus for stably providing similar cases.

The image display apparatus includes a display unit, a storage unit that stores a feature amount of a lesion candidate detected in a designated region of a breast image, the designated region being designated by a doctor in the breast image, in association with the breast image, a candidate detection unit that detects the lesion candidate in a designated region of a breast image to be diagnosed, the designated region being designated by a user, and calculates a feature amount of the lesion candidate, and a controller that acquires a breast image corresponding to a feature amount similar to the calculated feature amount from the storage unit and displays the breast image as a similar case on the display unit.

### SUMMARY OF THE INVENTION

However, in the techniques disclosed in JP2006-325640A and JP2010-000130A, the calcification is analyzed by analyzing the density gradient of the radiation image of the breast. Therefore, although it is possible to obtain an image of a small calcification lesion, there is a problem in that it is not possible to eliminate the influence of the difference from the background image due to the difference in the distribution of the mammary glands, the thickness of the breast, and the like, and the noise other than the calcification lesion still has a large influence.

The present disclosure has been made in view of the above circumstances, and an object of the present disclosure is to provide an image processing apparatus, a radiography system, and a program that can detect a calcification lesion from a radiation image of a breast in a state in which an influence of noise is reduced, as compared with the related art.

In order to achieve the above object, an image processing apparatus according to a first aspect of the present disclosure comprises at least one processor, in which the processor acquires a radiation image obtained by imaging a breast with radiation, normalizes a rate of change in a brightness gradient in the acquired radiation image using a variation degree that indicates a degree of variation of a local region in the rate of change of the brightness gradient, and detects a position of a calcification lesion in the radiation image by performing threshold value processing using a predetermined threshold value on the normalized rate of change of the brightness gradient.

An image processing apparatus according to a second aspect of the present disclosure, in the image processing apparatus according to the first aspect, the processor may be configured to create the synthesized two-dimensional image of the breast using a detection result of the position of the calcification lesion.

An image processing apparatus according to a third aspect of the present disclosure is the image processing apparatus according to the first aspect or the second aspect, in which the variation degree is a standard deviation or a variance of each local region in the rate of change in the brightness gradient.

An image processing apparatus according to a fourth aspect of the present disclosure is the image processing apparatus according to the first aspect or the second aspect, in which the processor performs the normalization by multiplying the reciprocal of the variation degree by the rate of change in the brightness gradient.

An image processing apparatus according to a fifth aspect of the present disclosure, in the image processing apparatus according to the first or second aspect, the processor may be configured to derive the rate of change in the brightness gradient by filtering using a gradient concentration filter.

An image processing apparatus according to a sixth aspect of the present disclosure, in the image processing apparatus according to the fifth aspect, the processor is configured to apply any one of a gradient concentration filter having isotropic weighting or a gradient concentration filter having anisotropic weighting.

In addition, in order to achieve the above object, a radiography system according to a seventh aspect of the present disclosure comprises the image processing apparatus according to the present disclosure, and a radiography apparatus that captures a radiation image used by the image processing apparatus.

Further, in order to achieve the above object, a program according to an eighth aspect of the present disclosure causes a computer to execute a process comprising: acquiring a radiation image obtained by imaging a breast with radiation; using a variation degree that indicates a degree of variation of a local region in a rate of change in a brightness gradient in the acquired radiation image to normalize the rate of change in the brightness gradient; and performing threshold value processing using a predetermined threshold value on the normalized rate of change in the brightness gradient to detect a position of a calcification lesion in the radiation image.

According to the present disclosure, it is possible to detect a calcification lesion from a radiation image of a breast in a state in which the influence of noise is reduced, as compared with the related art.

### BRIEF DESCRIPTION OF THE DRAWINGS

Fig. 1 is a schematic configuration diagram of a radiography system to which an image processing apparatus according to an embodiment is applied.
Fig. 2 is a diagram illustrating a mammography apparatus according to the embodiment as viewed from a direction of an arrow A in Fig. 1.
Fig. 3 is a block diagram illustrating an example of an electrical configuration of the image processing apparatus according to the embodiment.
Fig. 4 is a functional block diagram illustrating a functional configuration of the image processing apparatus according to the embodiment.
Fig. 5 is a diagram for describing acquisition of projection images according to the embodiment.
Fig. 6 is a diagram for describing generation of tomographic images according to the embodiment.
Fig. 7 is a diagram illustrating an example of a gradient concentration filter according to the embodiment and a method of deriving a rate of change in a brightness gradient by the gradient concentration filter for a radiation image.
Fig. 8 is a diagram for describing an effect of filtering by the gradient concentration filter according to the embodiment.
Fig. 9 is a diagram for describing a problem in a case where the normalization unit according to the embodiment does not perform normalization.
Fig. 10A is a diagram for explaining an effect of normalization by a normalization unit according to the embodiment.
Fig. 10B is a diagram for describing an effect of normalization by a normalization unit according to the embodiment.
Fig. 10C is a diagram for describing an effect of normalization by a normalization unit according to the embodiment.
Fig. 11 is a diagram illustrating an example of a synthesized two-dimensional image generated from a tomographic image group according to the embodiment.
Fig. 12 is a flowchart illustrating an example of image processing according to the embodiment.

### DESCRIPTION OF THE PREFERRED EMBODIMENTS

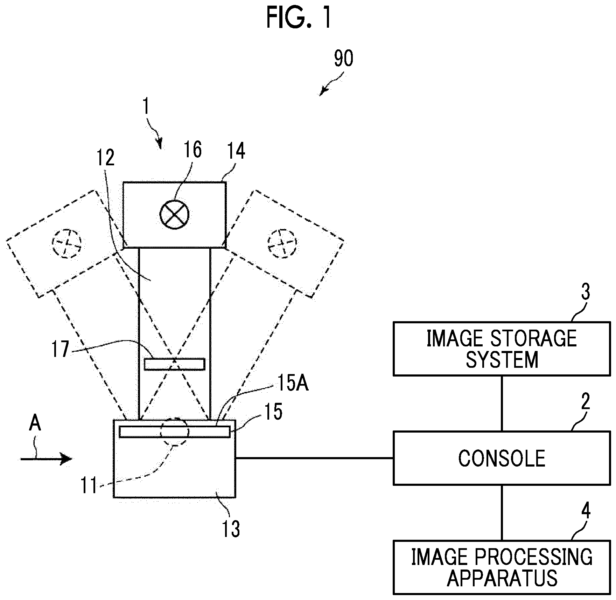

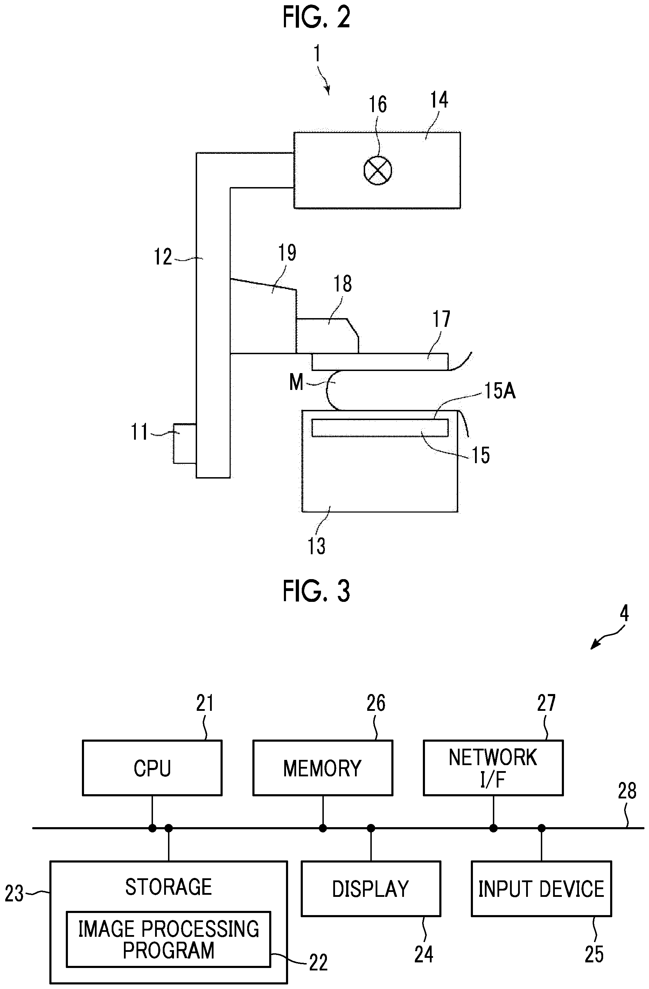

Hereinafter, embodiments of the present disclosure will be described with reference to the drawings. Fig. 1 is a schematic configuration diagram of a radiography system 90 to which an image processing apparatus 4 according to an embodiment of the present disclosure is applied, and Fig. 2 is a diagram illustrating a mammography apparatus 1 in the radiography system 90 as viewed from a direction of an arrow A in Fig. 1.

As illustrated in Fig. 1, a radiography system 90 according to the present embodiment performs imaging of a breast M, which is a subject, at a plurality of radiation source positions and acquires a plurality of radiation images, that is, a plurality of projection images, in order to perform tomosynthesis imaging on the breast to generate tomographic images. The radiography system 90 according to the present embodiment comprises a mammography apparatus 1, a console 2, an image storage system 3, and an image processing apparatus 4. The mammography apparatus 1 corresponds to a radiography apparatus according to the technology of the present disclosure.

The mammography apparatus 1 comprises an arm part 12 that is connected to a base (not illustrated) by a rotation shaft 11. An imaging table 13 is attached to one end of the arm part 12, and a radiation emitting unit 14 is attached to the other end of the arm part 12 to face the imaging table 13. The arm part 12 is configured such that only the end to which the radiation emitting unit 14 is attached can be rotated. Therefore, the imaging table 13 is fixed and only the radiation emitting unit 14 can be rotated.

A radiation detector 15, such as a flat panel detector, is provided in the imaging table 13. The radiation detector 15 has a radiation detection surface 15A. In addition, a circuit board including a charge amplifier that converts a charge signal read from the radiation detector 15 into a voltage signal, a sampling two correlation pile circuit that samples the voltage signal output from the charge amplifier, and an analog-to-digital (AD) conversion unit that converts the voltage signal into a digital signal is provided in the imaging table 13.

A radiation source 16 is accommodated in the radiation emitting unit 14. The radiation source 16 emits X-rays as radiation. The console 2 controls a timing when the radiation source 16 emits the radiation and radiation generation conditions of the radiation source 16, that is, selection of a target and filter materials, a tube voltage, an irradiation time, and the like.

Further, the arm part 12 is provided with a compression plate 17 that is disposed above the imaging table 13 and presses and compresses the breast M, a support part 18 that supports the compression plate 17, and a moving mechanism 19 that moves the support part 18 in a vertical direction in Fig. 1 and Fig. 2. The interval between the compression plate 17 and the imaging table 13, that is, the compression thickness is input to the console 2.

The console 2 has a function of controlling the mammography apparatus 1 using, for example, an imaging order and various kinds of information acquired from a radiology information system (RIS) (not illustrated) or the like through a network, such as a wireless communication local area network (LAN), and instructions or the like directly issued by a technician or the like. Specifically, the console 2 acquires a plurality of projection images as described below by causing the mammography apparatus 1 to perform tomosynthesis imaging of the breast M. As an example, in the present embodiment, a server computer is used as the console 2.

The image storage system 3 is a system that stores image data such as the projection images which are obtained by imaging of the mammography apparatus 1. The image storage system 3 extracts an image corresponding to a request from, for example, the console 2 and the image processing apparatus 4 from the stored images, and transmits the image to an apparatus that is the source of the request. A specific example of the image storage system 3 is a picture archiving and communication system (PACS).

Next, the image processing apparatus 4 according to the present embodiment will be described. Next, a hardware configuration of the image processing apparatus 4 according to the present embodiment will be described with reference to Fig. 3. As illustrated in Fig. 3, the image processing apparatus 4 is a computer, such as a workstation, a server computer, and a personal computer, and comprises a central processing unit (CPU) 21, a non-volatile storage 23, and a memory 26 as a transitory storage region. In addition, the image processing apparatus 4 comprises a display 24 such as a liquid crystal display, an input device 25 such as a keyboard and a mouse, and a network interface (I/F) 27 connected to a network (not illustrated). The CPU 21, the storage 23, the display 24, the input device 25, the memory 26, and the network I/F 27 are connected to a bus 28. The CPU 21 is an example of a processor according to the present disclosure.

The storage 23 is implemented by a hard disk drive (HDD), a solid state drive (SSD), a flash memory, and the like. An image processing program 22 to be installed in the image processing apparatus 4 is stored in the storage 23 as a storage medium. The CPU 21 reads out the image processing program 22 from the storage 23, expands the image processing program 22 in the memory 26, and executes the expanded image processing program 22.

The image processing program 22 is stored in a storage device of a server computer connected to the network or in a network storage in a state of being accessible from the outside, and is downloaded and installed in the computer that configures the image processing apparatus 4 in response to a request. Alternatively, the image processing program 22 is distributed in a state of being recorded on a recording medium, such as a digital versatile disc (DVD) or a compact disc read only memory (CD-ROM), and is installed in the computer that configures the image processing apparatus 4 from the recording medium.

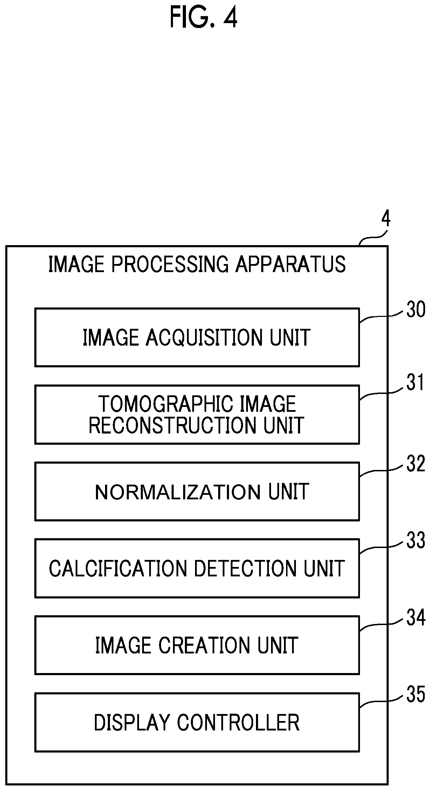

Hereinafter, a functional configuration of the image processing apparatus 4 according to the present embodiment will be described. Fig. 4 is a diagram illustrating a functional configuration of the image processing apparatus 4 according to the present embodiment.

As illustrated in Fig. 4, the image processing apparatus 4 according to the present embodiment comprises an image acquisition unit 30, a tomographic image reconstruction unit 31, a normalization unit 32, a calcification detection unit 33, an image creation unit 34, and a display controller 35. Then, the CPU 21 executes the image processing program 22 to function as the image acquisition unit 30, the tomographic image reconstruction unit 31, the normalization unit 32, the calcification detection unit 33, the image creation unit 34, and the display controller 35.

The image acquisition unit 30 acquires a plurality of projection images acquired by causing the console 2 to perform tomosynthesis imaging on the breast M by the mammography apparatus 1. The image acquisition unit 30 acquires the projection images from the console 2 or the image storage system 3 via the network I/F 27. Further, the tomographic image reconstruction unit 31 reconstructs a plurality of tomographic images from the projection images acquired by the image acquisition unit 30.

Here, the tomosynthesis imaging and the generation of the tomographic image will be described. In a case of performing the tomosynthesis imaging for generating a tomographic image, the console 2 acquires a plurality of projection images Gi (i = 1 to n, where n is the number of radiation source positions and is, for example, n = 15) at a plurality of radiation source positions by moving the radiation source 16 by rotating the arm part 12 around the rotation shaft 11, irradiating the breast M as a subject with radiation at a plurality of radiation source positions obtained by the movement of the radiation source 16 according to predetermined imaging conditions for tomosynthesis imaging, and detecting the radiation passing through the breast M by the radiation detector 15.

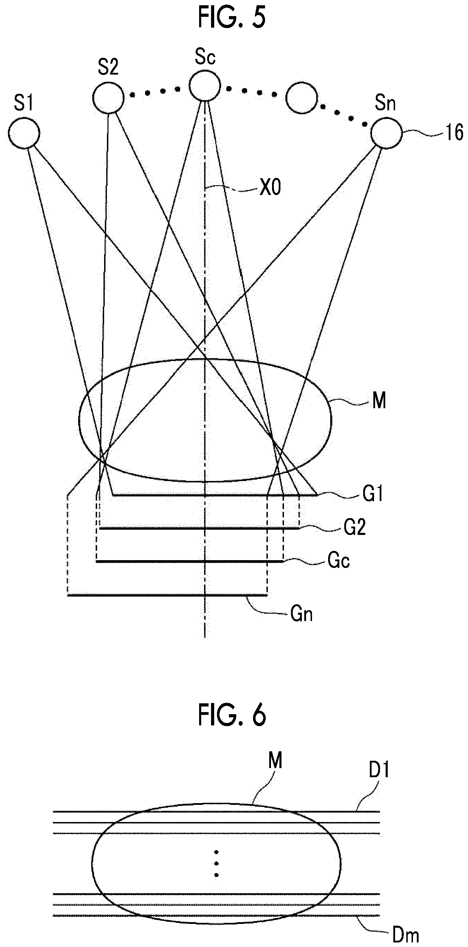

Fig. 5 is a diagram for describing the acquisition of the projection images Gi. As illustrated in Fig. 5, the radiation source 16 is moved to each of radiation source positions S1, S2, ···, and Sn. The radiation source 16 drives and irradiates the breast M with radiation at each of the radiation source positions. The radiation detector 15 detects X-rays passing through the breast M, and thus the projection images G1, G2, ···, and Gn corresponding to the radiation source positions S1 to Sn are acquired. At each of the radiation source positions S1 to Sn, the breast M is irradiated with the same dose of radiation.

In Fig. 5, the radiation source position Sc is a radiation source position at which the optical axis X0 of the radiation emitted from the radiation source 16 is orthogonal to the detection surface 15A of the radiation detector 15. The radiation source position Sc is referred to as a reference radiation source position Sc.

In addition, the tomographic image reconstruction unit 31 generates a tomographic image in which the desired tomographic planes of the breast M are emphasized by reconstructing the plurality of projection images Gi. Specifically, the image processing apparatus 4 generates a plurality of tomographic images Dj (j = 1 to m) on each of the plurality of tomographic planes of the breast M as illustrated in Fig. 6 by reconstructing the plurality of projection images Gi using a known back projection method, such as a simple back projection method or a filtered back projection method. In this case, a three-dimensional coordinate position in a three-dimensional space including the breast M is set, pixel values of the corresponding pixels in the plurality of projection images Gi are reconstructed with respect to the set three-dimensional coordinate position, and pixel values at the coordinate positions are calculated.

In the following, a case in which a plurality of tomographic images obtained by the above processing are applied as a "radiation image" of the technology of the present disclosure will be described. Therefore, in the following description, the term "tomographic image" may be referred to as a "radiation image" for convenience.

The normalization unit 32 normalizes the rate of change in the brightness gradient using a variation degree that indicates a degree of variation of each local region in the rate of change in the brightness gradient in the radiation image obtained by the above-described processing.

The normalization unit 32 according to the present embodiment applies the standard deviation for each local region in the rate of change in the brightness gradient as the variation degree, but the present invention is not limited to this form. For example, a form may be adopted in which the variance of each local region in the rate of change in the brightness gradient is applied as the variation degree. In short, any value may be applied as the variation degree according to the present embodiment as long as it is a value indicating the degree of variation of each local region in the rate of change in the brightness gradient.

In addition, the normalization unit 32 according to the present embodiment performs the normalization by multiplying the reciprocal of the variation degree by the rate of change in the brightness gradient. However, the present invention is not limited to this form, and any processing can be applied as long as the processing is a processing of standardizing the rate of change in the brightness gradient using the variation degree, for example, processing of dividing the rate of change in the brightness gradient by the variation degree.

In addition, the normalization unit 32 according to the present embodiment derives the rate of change in the brightness gradient by filtering using a gradient concentration filter. However, the present invention is not limited to this aspect, and a method in which the rate of change in the brightness gradient of the radiation image is derived by filtering with a filter other than the gradient concentration filter or a method in which the change rate of the brightness gradient of the radiation image is derived without using a filter can be applied as long as the rate of change in the brightness gradient of the radiation image can be derived.

In addition, the normalization unit 32 according to the present embodiment selectively applies any one of a filter having isotropic weighting or a filter having anisotropic weighting as the gradient concentration filter to derive the rate of change in the brightness gradient of the radiation image. In a case where the gradient concentration filter having isotropic weighting is applied, the detection effect is high for a small calcification close to a circle. On the other hand, in a case where the gradient concentration filter having anisotropic weighting is applied, the separation performance of the calcification having a long shape (shape of a long elliptical shape) in the direction component is high. Therefore, in the normalization unit 32 according to the present embodiment, the gradient concentration filter having isotropic weighting and the gradient concentration filter having anisotropic weighting are used according to the shape of the calcification lesion to be detected. The processing by the normalization unit 32 according to the present embodiment will be described in detail later.

On the other hand, the calcification detection unit 33 detects the position of the calcification lesion in the radiation image by performing threshold value processing using a predetermined threshold value with respect to the normalized rate of change in the brightness gradient. In the present embodiment, as the threshold value, a value statistically obtained in advance from an existing radiation image is applied as a value that can be considered as a pixel in which a calcification lesion is indicated in a pixel in which a rate of change in a brightness gradient equal to or greater than the threshold value is equal to or greater than the threshold value, but the present disclosure is not limited to this form. For example, a form may be adopted in which the threshold value is set in advance by being input in advance by the technician or the like according to the purpose of detecting the calcification lesion, the medical condition of the subject, and the like.

The image creation unit 34 creates a synthesized two-dimensional image of the subject (in the present embodiment, the breast M) using the position of the calcification lesion detected by the calcification detection unit 33.

In the image creation unit 34 according to the present embodiment, the synthesized two-dimensional image is created by performing a weighted average for each pixel using a weight value (for example, 0.9) of the tomographic image including the position of the calcification lesion detected by the calcification detection unit 33 as a value larger than a weight value (for example, 0.1) of a tomographic image other than the tomographic image. Here, the method of creating the synthesized two-dimensional image is not limited to such an averaging method, and an addition method, a maximum intensity projection method, a minimum intensity projection method, or the like may be applied as a method of creating a synthesized two-dimensional image.

For example, in a case where the minimum value projection method is applied as a method of creating the synthesized two-dimensional image, a form in which only the tomographic image including the position of the calcification lesion among the tomographic images to be projected is targeted can be exemplified.

As described above, in the present embodiment, the synthesized two-dimensional image is applied as the image created by the image creation unit 34, but the present disclosure is not limited thereto. For example, a form in which a slab image that is an image including thickness information may be applied as the image created by the image creation unit 34 may be adopted. As a form example in this case, a form in which a slab image is created using only the tomographic image including the position of the calcification lesion can be exemplified.

The display controller 35 displays the synthesized two-dimensional image generated by the image creation unit 34 on the display 24. The synthesized two-dimensional image is created by increasing the weight value for the tomographic image including the position of the calcification lesion, as compared with other tomographic images. Therefore, the synthesized two-dimensional image displayed here is an extremely high-quality image in which the calcification lesion is emphasized.

The image created by the image creation unit 34 is not limited to the synthesized two-dimensional image and the slab image described above, and an image for performing display of linking the synthesized two-dimensional image created from the plurality of tomographic images Dj with the corresponding tomographic images Dj may be applied.

That is, in general, the number of the tomographic images may be equal to or larger than 100 depending on the application. Therefore, in a case of reading the tomographic images, there is a problem that a relatively long reading time is required.

Therefore, in this form, the CPU 21 first creates a synthesized two-dimensional image as described above, and displays the synthesized two-dimensional image. Accordingly, an operator refers to the displayed synthesized two-dimensional image, and in a case where a calcification lesion is suspected in the structure of interest displayed on the synthesized two-dimensional image, designates the structure of interest. In response to the designation, the CPU 21 displays the tomographic image including the designated structure of interest. Thereby, a reading time of the tomographic image can be significantly shortened.

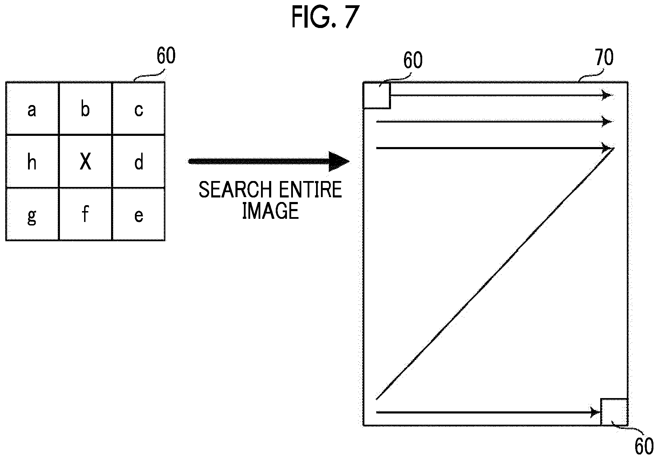

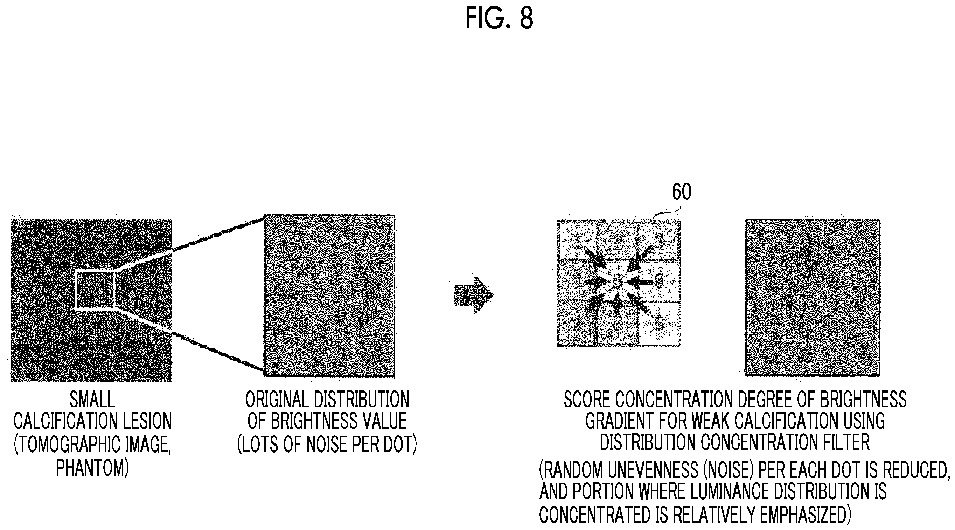

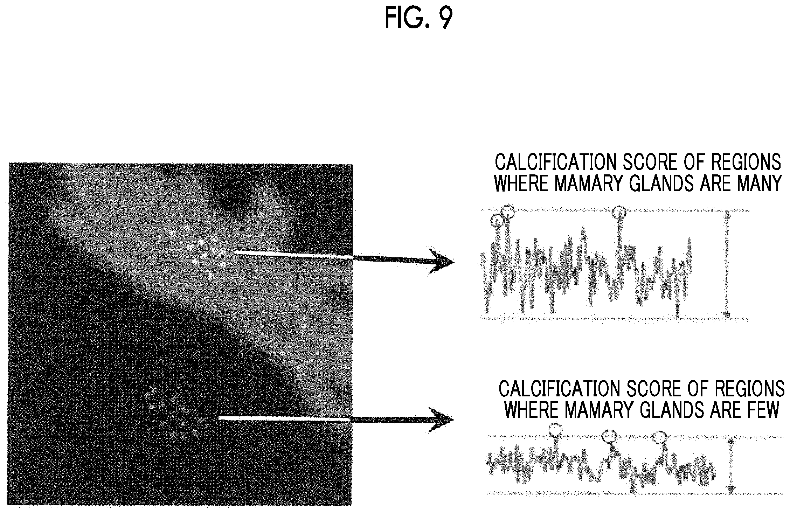

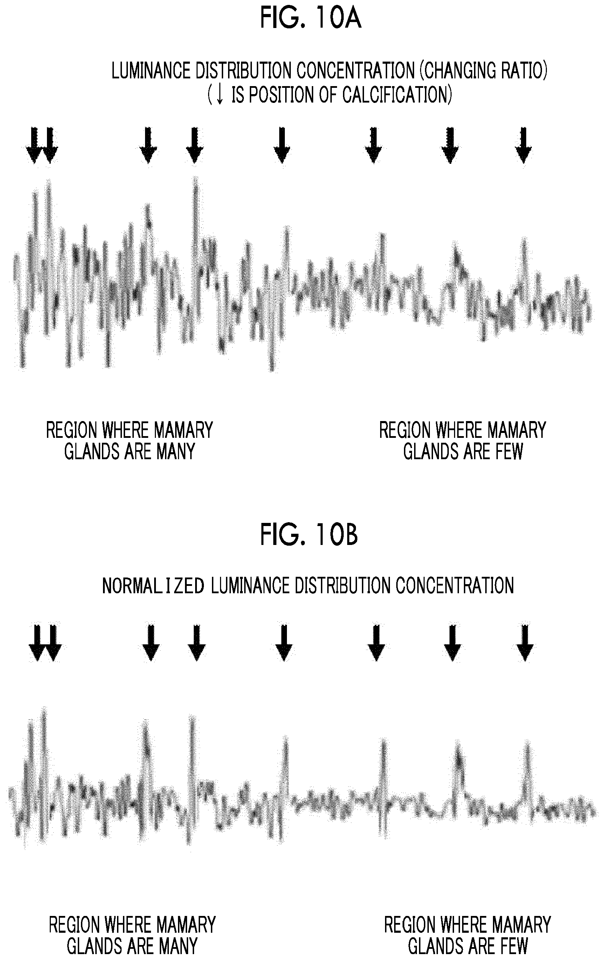

Next, a process by the normalization unit 32 according to the present embodiment will be specifically described with reference to Figs. 7 to 10. It should be noted that Fig. 7 is a diagram for describing an example of the gradient concentration filter 60 according to the present embodiment and a method of deriving a rate of change in a brightness gradient by the gradient concentration filter 60 for the radiation image 70. In addition, Fig. 8 is a diagram for describing the effect of filtering by the gradient concentration filter 60 according to the present embodiment. In addition, Fig. 9 is a diagram for describing a problem in a case where the normalization by the normalization unit 32 according to the present embodiment is not performed, and Figs. 10A to 10C are diagrams for describing an effect of the normalization by the normalization unit 32 according to the present embodiment.

In the normalization unit 32 according to the present embodiment, as an example, the rate of change in the brightness gradient of the radiation image is derived by filtering using a gradient concentration filter 60 having a size of 3 × 3 illustrated in the left diagram of Fig. 7. In the gradient concentration filter 60, in a case where the position of the center of the gradient concentration filter 60 is the attention pixel X and having anisotropic weighting, a rate of change Xc in the brightness gradient of the attention pixel X is derived by the following Equation (1) as an example. Xc = (X - a)/1.4 + (X - b)/1.4 + (X - c)/1.4 + (X - d) + (X - e)/1.4 + (X - f) + (X - g)/1.4 + (X - h)

In a case where a gradient concentration filter 60 having isotropic weighting is applied, all "1.4" in the right side of Expression (1) may be set to "1.0".

Then, in the normalization unit 32 according to the present embodiment, as indicated by an arrow in the right diagram of Fig. 7, the gradient concentration filter 60 is sequentially moved one row at a time toward the lower end portion of the radiation image 70 from the upper left corner point of the radiation image 70 to the right side. As a result, filtering using the gradient concentration filter 60 can be performed on all pixels of the radiation image 70.

As described above, in the present embodiment, the gradient concentration filter 60 having a size of 3 × 3 is applied, but the present disclosure is not limited to this form. For example, a form may be adopted in which a gradient concentration filter having a size other than the size of 3 × 3, such as a size of 5 × 5 or a size of 7 × 7, is applied as the gradient concentration filter 60.

The left diagram of Fig. 8 shows an example of a distribution of brightness values in a case where a small calcification lesion is reflected in a tomographic image generated for a phantom as a target. However, as shown in the diagram, the distribution of brightness values in this case has a lot of noise for each dot. On the other hand, as shown in the right diagram of Fig. 8, in the distribution of the brightness values that have been subjected to filtering using the gradient concentration filter 60, the random unevenness (noise) for each dot is attenuated, and a portion where the brightness gradient is concentrated is relatively emphasized.

However, in a case where the portion where the brightness gradient is concentrated is relatively emphasized only by the filtering by the gradient concentration filter 60, as shown in Fig. 9 as an example, there is a difference in the distribution of mammary glands in the tissue, the thickness of the breast for each subject, and the like. Therefore, the signal-noise ratio (SNR, SN ratio) varies. As a result, it is difficult to emphasize and display the calcification lesion in the synthesized two-dimensional image. The graph in the upper right in Fig. 9 is an example of the score of the calcification lesion in the region where the mammary glands are relatively many, and the graph in the lower right in Fig. 9 is an example of the score of the calcification lesion in the region where the mammary glands are relatively few. The "score" shown in Figs. 8 and 9 represents a rate of change in the brightness gradient, and substantially represents the intensity of the calcification.

In general, the calcification lesion has a size ranging from a relatively large size to a small size. However, in the image processing apparatus 4 according to the present embodiment, a small calcification lesion having a diameter of, for example, approximately 100 µm is a main detection target, and the influence of noise due to the variation in the SNR described above is serious.

Therefore, the normalization unit 32 according to the present embodiment derives a variation degree (in the present embodiment, a standard deviation) indicating a degree of variation of each local region in a rate of change in a brightness gradient in the radiation image by using a local moving kernel. Then, the normalization unit 32 according to the present embodiment normalizes the rate of change in the brightness gradient using the variation degree.

The "movement kernel" described herein indicates an area to be calculated, which is defined by the algorithm on the radiation image. In the present embodiment, for example, a window size of 31 pixels is defined in both the vertical direction and the horizontal direction, and the standard deviation is calculated in the area. The standard deviation in the area is calculated while moving the window for calculating the standard deviation having a size of 31 × 31 pixels from the upper left to the lower right of the radiation image one pixel at a time, as in the case of the gradient concentration filter 60 illustrated in the right diagram of Fig. 7. The standard deviation value obtained as a result of this calculation is held as a value at the center of the 31 × 31 window (position in the window is (15, 15)). With this processing, the standard deviation for each pixel of the radiation image can be obtained.

Then, in the normalization unit 32 according to the present embodiment, the rate of change in the derived brightness gradient is normalized by multiplying the derived rate of change in the brightness gradient by the reciprocal of the derived standard deviation for each corresponding pixel.

For example, in a case where a 31 × 31 window is present at a certain position of the derivation result of the brightness gradient, and the standard deviation at the certain position is 2.8 and the standard deviation at another position is 1.3, the normalization according to the present embodiment indicates that the value of the brightness gradient is divided by 2.8 and 1.3, respectively. By this normalization, the standard deviation of the entire derivation result of the brightness gradient approaches 1.0.

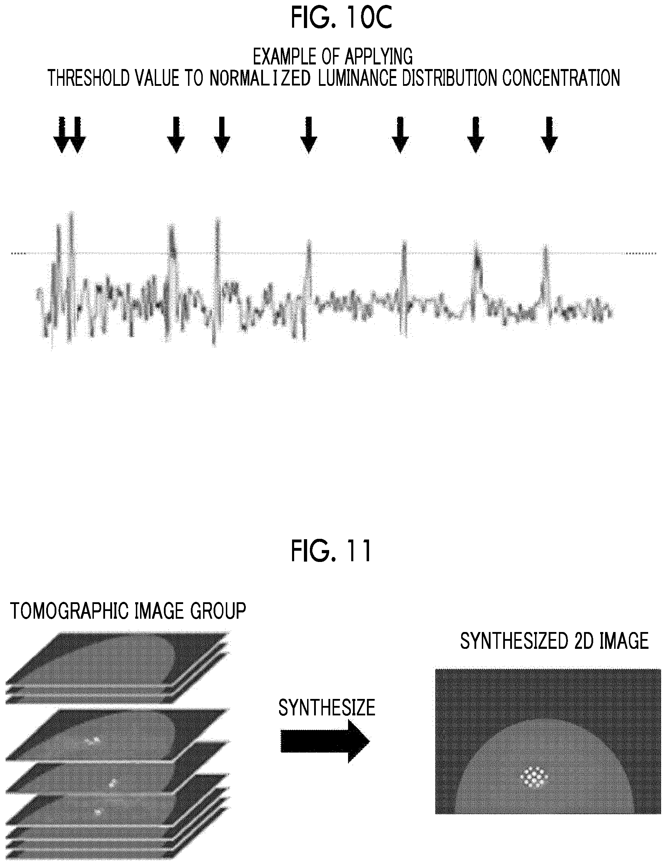

Figs. 10A to 10C show distributions of the rate of change in the brightness gradient from a region where the mammary glands are relatively many to a region where the mammary glands are relatively few, respectively. Fig. 10A shows the distribution before the normalization according to the present embodiment, Fig. 10B shows the distribution after the normalization, and Fig. 10C shows the distribution obtained by applying a threshold value to the distribution shown in Fig. 10B.

As illustrated in these figures, by performing the normalization according to the present embodiment, the influence of noise can be reduced. As a result, the small calcification lesion can be detected with high accuracy by the threshold value processing.

In addition, as shown in Fig. 11 as an example, by using the position of the calcification lesion detected by the threshold value processing to emphasize the position of the calcification lesion in the tomographic image group as described above to generate the synthesized two-dimensional image, it is possible to obtain a synthesized two-dimensional image in which a small calcification lesion is emphasized. It should be noted that Fig. 11 is a diagram showing an example of a synthesized two-dimensional image generated from the tomographic image group according to the present embodiment.

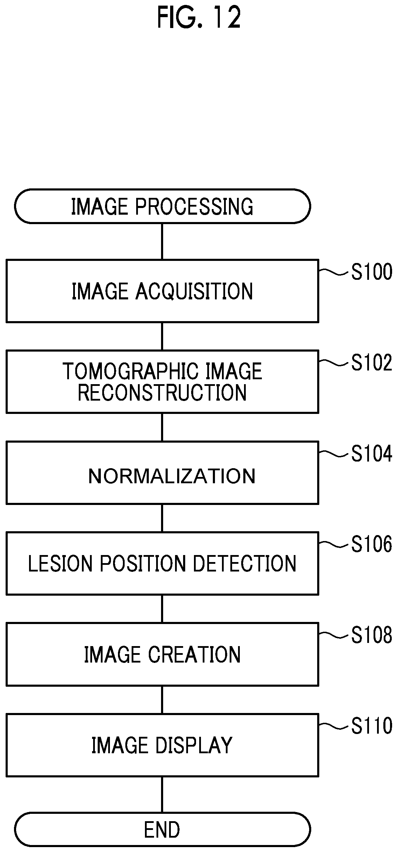

Next, processing performed in the present embodiment will be described. Fig. 12 is a flowchart illustrating image processing performed in the present embodiment. It is assumed that a plurality of projection images Gi are acquired in advance and stored in the storage 23. In addition, in order to avoid confusion, here, a case where any one of the filter having isotropic weighting or the filter having anisotropic weighting is selected in advance as the gradient concentration filter 60 applied in the main image processing will be described.

In a case where the input device 25 receives an instruction to start the processing from the operator, execution of the image processing program 22 is started, and thus, the image processing illustrated in Fig. 12 is started.

First, the image acquisition unit 30 acquires the projection images Gi by reading the projection images Gi from the storage 23, the projection images Gi being obtained by imaging the subject (in the present embodiment, the breast M) with the radiation emitted from the radiation source 16 at a plurality of angles (step S100). Next, the tomographic image reconstruction unit 31 reconstructs the plurality of tomographic images Dj from the acquired projection images Gi (step S102).

Next, the normalization unit 32 performs the above-described normalization processing on the plurality of reconstructed tomographic images Dj (step 104). Then, the calcification detection unit 33 detects the position of the above-mentioned calcification lesion using the normalized tomographic image Dj (step S106).

Then, the image creation unit 34 creates the synthesized two-dimensional image from the plurality of tomographic images Dj using the detected position of the calcification lesion as described above (Step 108). Subsequently, the display controller 35 displays the synthesized two-dimensional image on the display 24 (step S110), and the processing is ended. As a result of the processing of step S110, the synthesized two-dimensional image illustrated in the right diagram of Fig. 11 is displayed on the display 24.

As described above, with the image processing apparatus according to the present embodiment, the position of the calcification lesion in the radiation image is detected by acquiring the radiation image obtained by imaging the breast with radiation, standardizing the rate of change in the brightness gradient using the variation degree that indicates the degree of variation of the rate of change in the brightness gradient for each local region in the acquired radiation image, and performing the threshold value processing using the predetermined threshold value on the normalized rate of change in the brightness gradient. Therefore, it is possible to detect the calcification lesion from the radiation image of the breast in a state in which the influence of the noise is reduced, as compared with the related art.

In addition, with the image processing apparatus according to the present embodiment, the synthesized two-dimensional image of the breast is created using the detection result of the position of the calcification lesion. Therefore, it is possible to obtain the synthesized two-dimensional image in which the calcification lesion is emphasized in a state in which the influence of noise is reduced as compared with the related art.

In addition, with the image processing apparatus according to the present embodiment, the variation degree is a standard deviation or a variance of each local region in the rate of change in the brightness gradient. Therefore, the variation degree can be derived by standard deviation or variance, which is a statistical method.

In addition, with the image processing apparatus according to the present embodiment, the normalization is performed by multiplying the reciprocal of the variation degree by the rate of change in the brightness gradient. Accordingly, as a result of performing the normalization using the derived variation degree, it is possible to reduce the load of the operation as compared with a case where the normalization is performed using information other than the variation degree.

In addition, with the image processing apparatus according to the present embodiment, the rate of change in the brightness gradient is derived by filtering using the gradient concentration filter. Therefore, the rate of change in the brightness gradient can be easily derived by filtering.

Further, with the image processing apparatus according to the present embodiment, as the gradient concentration filter, any one of a filter having isotropic weighting or a filter having anisotropic weighting is selectively applied. Therefore, in a case where the gradient concentration filter having isotropic weighting is applied, the detection effect can be improved for the minute calcification close to a circle. In addition, in a case where the gradient concentration filter having anisotropy is applied with weighting, it is possible to improve the separation performance of a component in the direction of a long (long elliptical shape) shape of calcification, and thus, it is possible to contribute to the detection of a linear structure of a mammary gland and adipose tissue and a lesion such as an elliptical calcification that is likely to be buried therein.

In the embodiment, a case where the CPU 21 provided in the image processing apparatus 4 is applied as the processor according to the technology of the present disclosure has been described. On the other hand, the present disclosure is not limited thereto. For example, the CPU provided in any of the mammography apparatus 1, the console 2, and the image storage system 3 may be applied as a processor according to the technology of the present disclosure.

In addition, in the above-described embodiment, the case where the normalization of the technology of the present disclosure is applied to the tomographic image has been described, but the present disclosure is not limited thereto. For example, an aspect in which the normalization of the technology of the present disclosure is applied to a synthesized two-dimensional image may be adopted.

In addition, in the above-described embodiment, a case where any one of the filter having isotropic weighting or the filter having anisotropic weighting is selected in advance as the gradient concentration filter 60 to be applied has been described, but the present disclosure is not limited to this. For example, a form may be adopted in which the gradient concentration filter 60 is used in a plurality of stages, such as applying one gradient concentration filter 60 first and applying another gradient concentration filter 60 in a case where the calcification lesion is desired to be observed from another viewpoint.

In addition, in the embodiment, for example, as hardware structures of processing units that execute various kinds of processing, such as the image acquisition unit 30, the tomographic image reconstruction unit 31, the normalization unit 32, the calcification detection unit 33, the image creation unit 34, and the display controller 35, the following various processors can be used. The various processors include, in addition to a CPU that is a general-purpose processor functioning as various processing units by executing software (program) as described above, a programmable logic device (PLD) such as a field programmable gate array (FPGA) that is a processor having a circuit configuration changeable after manufacture, a dedicated electric circuit such as an application specific integrated circuit (ASIC) that is a processor having a circuit configuration dedicatedly designed to execute specific processing, and the like.

One processing unit may be composed of one of the various processors or may be composed of a combination of two or more processors of the same type or different types (for example, a combination of a plurality of FPGAs or a combination of a CPU and an FPGA). In addition, a plurality of processing units may be composed of one processor.

As an example of the plurality of processing units composed of one processor, first, the present disclosure includes an aspect in which one processor is composed of a combination of one or more CPUs and software, and the processor functions as the plurality of processing units, as represented by a computer such as a client and a server. Second, the present disclosure includes an aspect of using a processor that implements functions of the entire system including the plurality of processing units in one integrated circuit (IC) chip, as represented by a system on chip (SoC). In this way, various processing units are configured by one or more of the above-described various processors as hardware structures.

Furthermore, more specifically, an electric circuit (circuitry) in which circuit elements such as semiconductor elements are combined can be used as the hardware structure of the various processors.

In addition, in the embodiment described above, the aspect in which the image processing program 22 is stored (installed) in advance in the storage 23 of the image processing apparatus 4 has been described, but the present disclosure is not limited thereto. The image processing program 22 may be provided in a form recorded in a recording medium such as a compact disc read-only memory (CD-ROM), a digital versatile disc read-only memory (DVD-ROM), and a universal serial bus (USB) memory. In addition, the image processing program 22 may be configured to be downloaded from an external apparatus via a network.

The invention according to appendices below can be perceived from the above description.

### Supplementary Note 1

An image processing apparatus including: at least one processor, in which the processor is configured to: acquire a radiation image obtained by imaging a breast with radiation; normalize a rate of change in a brightness gradient in the acquired radiation image, using a variation degree that indicates a degree of variation of a local region in the rate of change in the brightness gradient; and detect a position of a calcification lesion in the radiation image by performing threshold value processing using a predetermined threshold value on the normalized rate of change in the brightness gradient.

### Supplementary Note 2

The image processing apparatus according to Supplementary Note 1, in which the processor is configured to: create the synthesized two-dimensional image of the breast using a detection result of the position of the calcification lesion.

### Supplementary Note 3

The image processing apparatus according to Supplementary Note 1 or 2, in which the variation degree is a standard deviation or a variance for each local region in a rate of change in the brightness gradient.

### Supplementary Note 4

The image processing apparatus according to any one of Supplementary Notes 1 to 3, in which the processor is configured to: perform the normalization by multiplying an inverse of the variation degree by a rate of change in the brightness gradient.

### Supplementary Note 5

The image processing apparatus according to any one of Supplementary Notes 1 to 4, in which the processor is configured to: derive the rate of change in the brightness gradient by filtering using a gradient concentration filter.

### Supplementary Note 6

The image processing apparatus according to Supplementary Note 5, in which the processor is configured to: apply any one of a gradient concentration filter having isotropic weighting or a gradient concentration filter having anisotropic weighting as the gradient concentration filter.

### Supplementary Note 7

A radiography system comprising: the image processing apparatus according to any one of Supplementary Notes 1 to 6; and a radiography apparatus that captures a radiation image used by the image processing apparatus.

### Supplementary Note 8

A program causing a computer to execute a process comprising: acquiring a radiation image obtained by imaging a breast with radiation; standardizing a rate of change in a brightness gradient in the acquired radiation image using a variation degree that indicates a degree of variation of a local region in the rate of change in the brightness gradient; and detecting a position of a calcification lesion in the radiation image by performing threshold value processing using a predetermined threshold value on the normalized rate of change in the brightness gradient.

## Claims

1. An image processing apparatus (4) comprising:
a processor (21),
wherein the processor (21) is configured to:
acquire a radiation image (70) obtained by imaging a breast (M) with radiation;
normalize a rate (Xc) of change in a brightness gradient in the acquired radiation image (70), using a variation degree that indicates a degree of variation of a local region in the rate (Xc) of change of the brightness gradient; and
detect a position of a calcification lesion in the radiation image (70) by performing threshold value processing using a predetermined threshold value on the normalized rate (Xc) of change in the brightness gradient.

2. The image processing apparatus (4) according to claim 1,
wherein the processor (21) is configured to:
create a synthesized two-dimensional image of the breast (M) using a detection result of the position of the calcification lesion.

3. The image processing apparatus (4) according to claim 1 or 2,
wherein the variation degree is a standard deviation or a variance for each local region in the rate (Xc) of change in the brightness gradient.

4. The image processing apparatus (4) according to claim 1 or 2,
wherein the processor (21) is configured to:
perform the normalization by multiplying an inverse of the variation degree by the rate (Xc) of change in the brightness gradient.

5. The image processing apparatus (4) according to claim 1 or 2,
wherein the processor (21) is configured to:
derive the rate (Xc) of change in the brightness gradient by filtering using a gradient concentration filter (60).

6. The image processing apparatus (4) according to claim 5,
wherein the processor (21) is configured to:
apply any one of a gradient concentration filter having isotropic weighting or a gradient concentration filter having anisotropic weighting as the gradient concentration filter (60).

7. A radiography system (90) comprising:
the image processing apparatus (4) according to claim 1 or 2; and
a radiography apparatus (1) that captures a radiation image (70) used by the image processing apparatus (4).

8. A storage medium (23) storing a program (22) executable by a computer to execute a process comprising:
acquiring a radiation image (70) obtained by imaging a breast (M) with radiation;
standardizing a rate (Xc) of change in a brightness gradient in the acquired radiation image (70) using a variation degree that indicates a degree of variation of a local region in the rate (Xc) of change in the brightness gradient; and
detecting a position of a calcification lesion in the radiation image (70) by performing threshold value processing using a predetermined threshold value on the normalized rate (Xc) of change in the brightness gradient.
